# EUROPEAN PATENT APPLICATION

(11) **EP 2 161 330 A1**
(43) Date of publication of application: **10.03.2010**
(21) Application number: 08290808.8
(22) Date of filing: 28.08.2008
(51) Int. Cl.: C12N 5/00

(54) **Experimental models of age-related macular degeneration and related retinal disorders**

(71) Applicant: Centre National de la Recherche Scientifique- CNRS, 75794 Paris Cedex 16 (FR); UNIVERSITE LOUIS PASTEUR, 67000 Strasbourg (FR)
(72) Inventor: Pfrieger, Frank W., F-67800 Bischheim (FR); Claudepierre, Thomas, F-67000 Strasbourg (FR)
(74) Representative: Novagraaf IP

(57) **Abstract**

The present invention relates to experimental models of age-related macular degeneration (AMD) and related retinal disorders and to screening methods using these models for detecting molecule candidates for the treatment of AMD. In particular, the present invention relates to the use of an animal or a cell that is deficient in functional Niemann Pick type C-related protein as experimental model of AMD and vision disorders resulting from AMD. The present invention founds industrial applications for preclinical research and development on age-related macular degeneration and similar diseases. Examples of potential industrial application are in companies that have a preclinical R&D program in retinal diseases, particularly in AMD.

## Description

### Technical field of the invention

This invention is related to the area of Age-Related Macular Degeneration (AMD), in particular to experimental models of AMD and related retinal disorders and to preclinical screening methods using these models to detect and validate drug candidates for the treatment of AMD.

The present invention founds industrial applications in drug discovery and preclinical research and development on age-related macular degeneration and similar diseases.

Examples of industrial applications are in pharmaceutical or biotechnology companies that have a preclinical R&D program in retinal diseases, particularly in AMD.

References indicated by "[ref. X]" refer to the Bibliography part after the example parts and before the claims.

### Background of the invention

AMD is a leading cause of vision loss affecting 30 million individuals worldwide. Since the risk to develop AMD increases at ages beyond 60, the prevalence of this disease rises rapidly in industrialized countries with aging populations and AMD will impose a profound socio-economic impact [Ref. 1].

AMD gradually destroys sharp vision by the macula resulting in the inability to read, to see fine details and to recognize faces.

Early hallmarks of AMD are the occurrence of lipofuscin granula and drusen in the retinal pigment epithelium (RPE) layer, which both are composed of incompletely defined lipids and proteins [Ref. 1].

Advanced AMD, which entails profound impairment of vision, is separated in two classes [Ref. 2]. The so-called dry form, which concerns the large majority of patients, results from atrophy in the RPE layer, which then causes loss of photoreceptors in the macula. Neovascular or wet AMD presents clinical signs of the dry form plus growth of new blood vessels from the choroid plexus through Bruch's membrane in the subretinal space, which can ultimately result in loss of vision.

Currently, there is no cure for any form of AMD.

There are a few drugs and treatments that delay the progression of the wet form and improve vision, which target blood vessels and stop their growth (Lucentis®, Macugen®) or reduce their leakiness (Photodynamic Therapy with Visudyne®).

Importantly, there is no treatment for the more prevalent dry form.

A major drawback for preclinical R&D on AMD is the lack of suitable experimental models that mimick the symptoms of AMD and that serve to test and validate drug candidates.

Mouse retinae represent an established animal model for retinal diseases, as they resemble the human version in fundamental physiological and anatomical aspects. So far, however, there is only a very limited number of mouse strains that develop clinical signs of AMD including drusen, lipofuscin and photoreceptor degeneration [Ref. 2]. Unfortunately, each of the mouse strains or lines mimics only a subset of the pathological changes of AMD and they appear at a rather late age. This late occurrence renders preclinical studies very costly and time-consuming.

Due to these disadvantages, there is an urgent need for new experimental models that recapitulate more AMD-related symptoms at younger ages and that can be used for preclinical R&D on this disease.

### Description of the invention

In one aspect, the present invention proposes the use of animals or cells that are deficient in functional Niemann Pick type C1 (NPC1) and related proteins as experimental models of age-related macular degeneration (AMD) and vision disorders resulting from AMD.

The experimental models mimic clinical signs of AMD and can therefore be used for preclinical research and development on efficient therapies for this disease. They include cell lines, primary cells, retinal explant cultures and animals.

In one embodiment of the present invention, said animal or cell is an animal or cell showing accumulation of lipids in the endosomallysosomal system. Said accumulation may be generated by a mutation in a NPC gene selected from the group comprising NPC1, NPCL1 and NPC2 genes. Said accumulation may be induced for example by a spontaneous or induced mutation of encoding DNA, by downregulation of mRNA or by pharmacological treatment. Animals carrying a spontaneous mutation in NPC1 are already available. In 1980, a mouse strain was discovered that showed a similar phenotype as Niemann-Pick type C patients (BALB/c npc^{nih}) [Ref. 4]. A subsequent study showed by linkage mapping and candidate gene analysis that this strain harbors a spontaneous mutation in the gene encoding for NPC1 [Ref. 5]. Induced mutations in NPC1 can be accomplished using the Cre/loxP system [Ref. 6]. This approach has the additional advantage that NPC1 can be ablated in selected retinal cell-types. To this end, transgenic mice are created with a targeted mutation of NPC1, where a functionally critical exon of NPC1 is flanked by loxP recognition sites of Cre recombinase. The wildtype allele of NPC1 in the mouse genome would be replaced by the conditional allele using homologous recombination in embryonic stem cells. A protocol to obtain such mice follows standard procedures that are described for example in detail [Ref. 7]. To accomplish genetic ablation of NPC1, mice homozygous for the conditional allele will be crossed with transgenic mice, which express Cre recombinase under the control of a promoter that drives gene expression in retinal cells including photoreceptors or RPE cells. Appropriate transgenic mouse lines have been generated [Refs. 8, 9]. Down-regulation of NPC1 in retinal cells or cell lines can be accomplished by transfection with RNA interference constructs according to a published protocol [Ref. 10]. Alternatively, a similar cellular phenotype as observed in NPC1-deficient animals can be induced pharmacologically by treatment of cells with U18666A (3-beta-[2-(diethylamino)ethoxy]androst-5-en-17-one) [Ref. 11, 12].

According to the present invention, said animal may be a mouse or a rat or any suitable animal, which is homozygous for a mutant allele of NPC1, for example a mouse from the BALB/cNctr-Npc1m1N/J strain, which is available from Jackson Labs (stock number 003092).

According to the present invention, when the model is based on cells, said cells may be cells or cell lines derived from the RPE or any other type of cells or cell lines showing phagocytotic activity, for example macrophages.

According to the present invention, when the model is based on cells, said cells with phagocytotic activity are preferably cultured together with entire photoreceptors or with outer segments (OS) of photoreceptors.

According to the present invention, when the model is based on cells, said cells with phagocytotic activity are characterized by the fact that they lack functional NPC or related proteins (NCP1, NPC1L1 or NPC2) or that cells lack proteins or overexpress proteins, whose absence or abundance causes similar changes as for NPC, respectively.

Alternatively, according to the present invention, when the model is based on cells, said cells may be obtained by any suitable treatment with chemical and/or physical substance(s) that modify intracellular transport of cholesterol and other lipids. For example, this chemical substance may be U18666A (3-beta-[2-(diethylamino)ethoxy]androst-5-en-17-one). This is an example of a chemical substance that has been shown to mimic the cellular NPC1 phenotype, namely accumulation of cholesterol in the endosomal/lysosomal system [Ref. 11, 12].

The models of AMD proposed in the present invention are based on a study of a mouse strain that lacks NPC1, a protein involved in intracellular cholesterol trafficking [Ref. 13]. This study was motivated by the working hypothesis that neuronal development and function depend on an exchange of cholesterol between neurons and glial cells [Ref. 14, 15].

The present inventors found that NPC1-deficient mice develop several symptoms similar to those of AMD within one and two months of age. These symptoms are histopathological changes in the retina that occur also in AMD patients. The similarity to AMD is a surprising observation, since a retinal phenotype of NPC1 deficiency has not been described previously.

The working hypothesis is that AMD-related histopathological changes in NPC1-deficient mouse are caused by accumulation of cholesterol and other lipids in the endosomal/lysosomal system, particularly in cells of the RPE. The idea that impaired cellular disturbed cholesterol trafficking plays a role in AMD is further supported by previous studies. Cholesterol accumulation between the RPE and Bruch's membrane has been detected in eyes of AMD patients [Ref. 16] and the lipofuscin component A2E, which has been associated with AMD, has been shown to perturb cholesterol trafficking in RPE cells [Ref. 17].

The fast onset of AMD-related retinal changes in NPC-deficient mouse appears as a distinctive property of this model. The experimental models of the present invention may comprise manipulations of NPC or related proteins (e.g. NCP1, NPC1L1 and NPC2) or other manipulations that cause the same outcome in animals, in retinae in vivo or ex vivo or in cells in vitro for their use in preclinical development of drugs to diagnose, prevent, limit or cure AMD. The deficiency or overexpression of NPC and related components (e.g. NCP1, NPC1L1 and NPC2) can be permanent or transient and can be induced by pharmacological or genetic modifications at the DNA or RNA level.

The in vitro experimental models that may be used according to the present invention contain cells with phagocytotic activity, for example cells from the RPE or macrophages, and photoreceptors or photoreceptor OS, which are ingested by RPE cells or macrophages.

As mentioned above, advanced AMD is separated in two classes [Ref. 2]. The "dry form", which concerns the large majority of patients, and neovascular or "wet form". The AMD models of the present invention are models for all forms of AMD.

In another aspect, the present invention provides a screening method of candidate molecules for the treatment of AMD. This screening method is usable for example for preclinical testing of drugs to treat AMD.

Screening methods may be applied onto cell- or animal-based models of the present invention.

Screening methods usable according to the present invention onto mouse models are disclosed in the Examples part. Any suitable method known by the skilled person may be used.

Screening methods usable according to the present invention onto cell models are disclosed in the Examples part. Any suitable method known by the skilled person may be used.

The cell- and animal-based models of the present invention provide new tools for preclinical development of drugs for the treatment or cure of AMD. They have two advantages compared to existing models: The animal-based model allows to test for effects of drug candidates on a more complete set of pathological changes than the existing animal models. The cell-based model enables drug candidate validation at an early step of preclinical drug development. Both models allow for faster and more cost-efficient development.

The models of the present invention may also be used for preclinical R&D for Niemann-Pick type C disease.

A complete understanding will be obtained by reference to the following specific examples, which are provided herein for purposes of illustration only, in the light of the annexed figure.

### Brief description of the figures

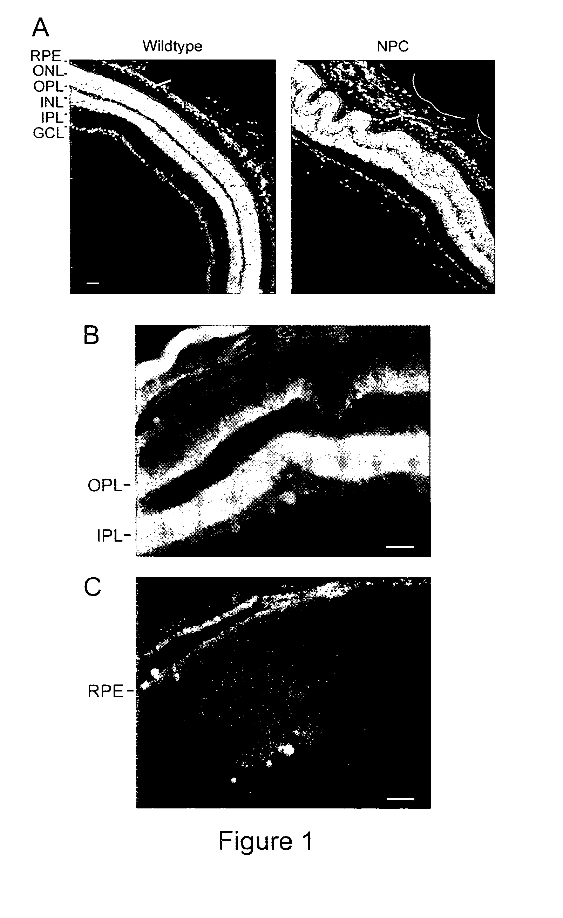

- Figure 1 shows histopathological changes in retinae of NPC mice. A, fluorescence micrographs of retinae from two months old wildtype (left) and NPC1 mutant animals mice stained with nuclear marker to reveal their laminated structure. Scale bar: 50 µm. B, Fluorescence micrograph of a retina from a two months old NPC1 mutant mouse after immunohistochemical staining for synaptophysin, which shows breaks in the outer plexiform layer and indicates defects in retinal synaptic transmission. C, Fluorescence micrograph of a retina from a two months old NPC1 mutant mouse stained with filipin, which reveals accumulation of cholesterol at the level of the RPE. Scale bars in B and C: 40 µm.

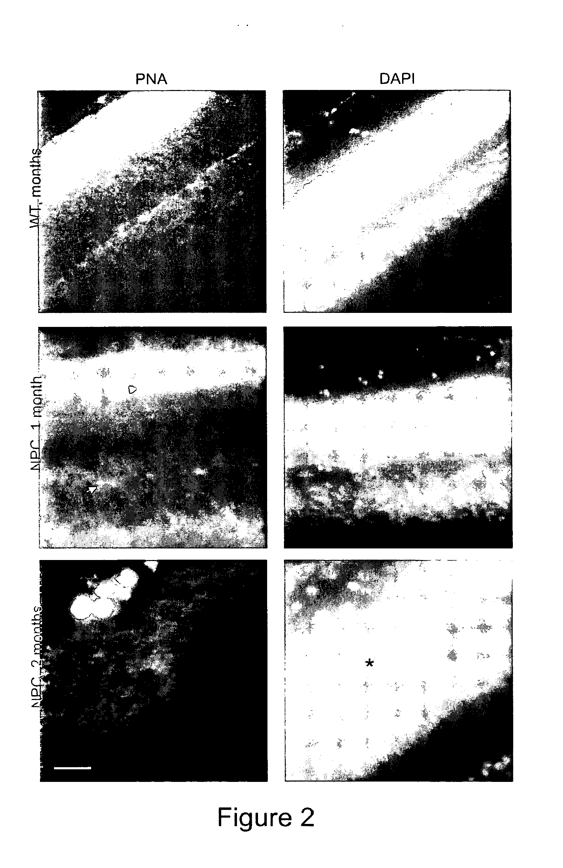

- Figure 2 shows progressive degeneration of outer segments and cone pedicles in NPC mice. Fluorescence micrographs of retinae from 2 months old wildtype (top), and one (middle) and two (bottom) months old NPC mutant mice stained with fluorescein-coupled peanut lectin (left) and with DAPI to visualize nuclei (right). Scale bar 50 µm.

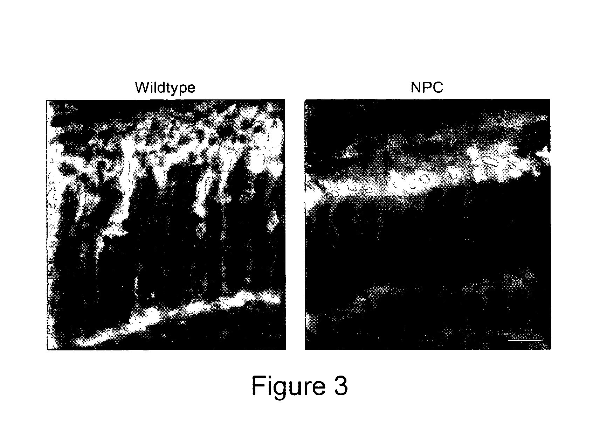

- Figure 3 shows degeneration of cones in NPC mice. Fluorescence micrographs of retinae from 2 months old wildtype and NPC1-deficient mice stained with an antibody against cone-specific opsin. Scale bar, 30 µm.

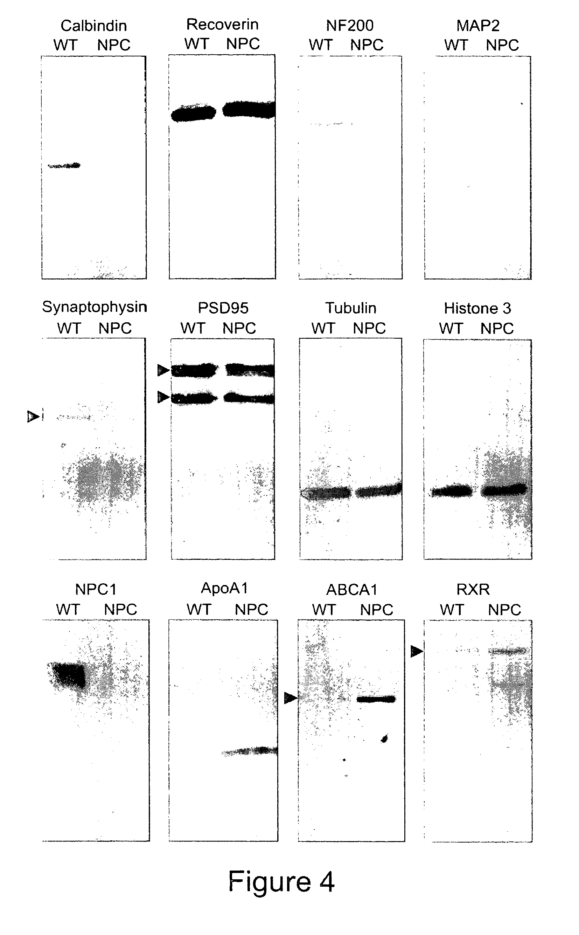

- Figure 4 shows immunoblots of retinae from wildtype and NPC1 mutant mice that were reacted with antibodies against indicated proteins including cellular, axonal/dendritic and synaptic markers and components involved in cholesterol homeostasis. Triangles indicate relevant protein bands.

### EXAMPLES

### Example 1 : Example of a mouse line lacking functional NPC1

This mouse represents a well-established animal model for NPC disease. The inventors' analysis revealed pathological changes that resemble hallmarks of AMD and the following example demonstrates that this mouse is usable to carry out the present invention.

The mouse of this example is the mouse line BALB/cNctr-Npc1 m1 N/J, Jackson Laboratory - reference number 003092.

### A. Histopathological changes

Analysis of histopathological changes of retinae of different mice have been carried out using the following protocols.

### A.1 Protocol used in this example for the analysis of histopathological changes in retinae of NPC mice by immunohistochemical staining.

Adult mice were killed by cervical dislocation. After enucleation, mouse eyeballs were fixed with 4% formaldehyde in PBS for 30 minutes and then incubated in increasing sucrose gradients (5, 15 and 30% sucrose in PBS). Eyeballs were then embedded in OCT compound and frozen in liquid nitrogen. They were sliced at 10 micrometer thickness in a cryostat and placed on gelatin-coated glass slides. The sections were blocked and permeabilized with 0.1 % Triton, 0.1 % BSA diluted in PBS for 15 min. They were incubated overnight with primary antibodies. Antibodies against synaptophysin (1:200; mouse, #S5768, Sigma) and opsin red/green (1:200; rabbit, AB5405, Chemicon/Millipore) were used. OS were labelled with FITC-coupled peanut lectin (PNA; 1:300; #L-21409; Molecular Probes/Invitrogen). Following incubation with primary antibodies, sections were washed three times with PBS. Secondary antibodies (Molecular Probes Eugene, OR) coupled to either Alexafluor 488 or 555 were used, diluted 1:500 in PBS. Sections were rinsed three times with PBS containing 0.1 % Tween 20. Cell nuclei were stained using DAPI at 1:200 in PBS for 5 minutes (Molecular Probes). Sections were examined on a fluorescence microscope (x20 and 40x objectives) (Optiphot 2; Nikon, Tokyo, Japan).

### A.2 Protocol used in this example for the analysis of cholesterol distribution by histochemical staining

To determine the distribution of cholesterol in retinae, mouse retinae were fixed (4% formaldehyde for 30 min at room temperature) and incubated with filipin (10 µg ml-1 for 2 h at room temperature, Sigma), an antibiotic that selectively binds to sterols with a hydroxy group at the 3rd carbon atom. Filipin fluorescence was then excited by monochromatic light (356 nm, Xenon lamp; Polychrome Junior, TILL Photonics, Munich, Germany), viewed on an upright microscope (Axioskop II FS, Zeiss) by an appropriate emission filter (Filter Set 2, Zeiss) and a 40 × objective and digitized by an air-cooled monochrome CCD camera (PCO Computer Optics, Kehlheim, Germany).

Annexed figures 1-3 show the experimental results obtained by using the above-disclosed protocols A1 and A.2.

In figure 1: RPE = retinal pigmented epithelium; ONL = outer nuclear layer; OPL = outer plexiform layer; INL = inner nuclear layer; IPL = inner plexiform layer; GCL = ganglion cell layer. Scale bars in A, 50 µm; in B and C, 40 µm.

Part A of annexed figure 1 shows fluorescence micrographs of retinae from two months old wildtype (left) and NPC1 deficient mice (right) stained with the nuclear marker DAPI to reveal the retinal layers. The laminated structure of the retina with the RPE layer arrows, left) was perturbed in NPC mice (right) and showed folds of the outer nuclear layer (ONL) and displaced nuclei that appear to correspond to RPE cells in the center of the folds (arrows, right). Retinae of two months old NPC1 mutant mice also showed distruptions of the outer plexiform layer (OPL) as indicated by immunostaining for synaptophysin (B) and detachment of RPE and Bruch's membrane (asterisk in C).

Given that the NPC mouse show massive accumulation of cholesterol in the endosomal/lysosomal system, retinae were stained with filipin, a fluorescent antibiotic that binds sterols. Filipin staining of retinae from 2 months old NPC mouse revealed accumulation of cholesterol along the RPE layer and occasionally in the ganglion cell layer (figure 1 C). Cholesterol clusters seen in annexed figure 1 may correspond to autofluorescent material seen in figure 2 suggesting that cholesterol is a component of the lipofuscin-like inclusions that accumulate during the second month of age.

Inventors' analysis of retinae of the mouse line BALB/cNctr-Npc1m1N/J revealed histopathological changes in two months (n = 8 retinae), but not in one month (n = 5 retinae) old mutant animals compared to wildtype littermates (2 months: n = 4).

In figure 2: WT, wildtype mice; NPC, NPC1 mutant mice. PNA, peanut agglutinin. Scale bar 50 µm.

Annexed figure 2 shows progressive degeneration of OS and cone pedicles in NPC1-deficient mice: fluorescence micrographs of retinae from two months old wildtype (top) and one (middle) and two (bottom) months old NPC1 mutant mice stained with fluorescein-coupled peanut lectin (left) and with DAPI (right) to visualize nuclei. One month old NPC mice showed normal retinal lamination, but beginning disorganization of OS (arrowhead, middle) and a lower density of cone pedicles (arrow) compared to wildtype mice. In two months old mutant mice, OS were absent (bottom, left) from folds of the outer nuclear layer (asterisk; bottom, right) and cone pedicles were absent from the underlying outer plexiform layer (bottom, left).

Annexed figure 3 shows degeneration of cones in NPC mice. Fluorescence micrographs of retinae from two months old wildtype and NPC mutant mice stained with an opsin antibody that labels cone photoreceptors. Note the reduced length and atrophied appearance of cones in NPC compared to wildtype mice. Scale bar, 30 µm.

Peanut agglutinin staining revealed progressive disorganization of OS in 1 and 2 months old animals (figure 2) and staining with an opsin antibody revealed a shortening of cone OS in two months old NPC1-deficient mice compared to wildtype littermates (figure 3).These results suggest that the recycling of photoreceptor OS is defective in the NPC mouse.

Notably, both, peanut agglutinin (figure 2) and opsin staining (figure 3) revealed a loss of cone pedicles, which confirmed the disruption of synaptic connections in the OPL, as indicated by synaptophysin staining as represented in annexed figure 1. Notably, a similar focal reduction of synaptic markers at photoreceptor tips has also been described in AMD patients in the vicinity of drusen accumulations [Ref. 18].

### B. Changes in protein levels of cellular markers, synaptic and cytoskeletal components and of components mediating cholesterol/lipid transport in retinae from NPC1 mutant mice.

Analysis of protein levels in retinae from wildtype and NPC1-deficient mice have been carried out using the following protocols.

### B.1 Protocol used in this example for the analysis of retinal protein expression in retinae from wildtype and NPC1 mutant mice

Mice were killed by cervical dislocation. For protein extraction, retinae were homogenized in 10 volumes extraction buffer containing 150 mM NaCl, 10 mM EDTA, 1% protease inhibitor (Sigma), 1 mM phenylmethylsulfonyl fluoride, and 25 mM Tris-HCl, pH 7.4. After centrifugation at 1,000g for 10 minutes, supernatants were recovered and centrifuged for an additional 30 minutes at 17000g, and the final enriched membrane fraction pellet was resuspended in extraction buffer.

Protein concentration was determined by means of the BCA assay (Pierce, Rockford, IL) according to the manufacturer's instructions. For immunoblots, twenty micrograms of each protein extract were separated by SDS-PAGE and then electrotransferred to nitrocellulose membranes (Bio-Rad, Hercules, CA).

The efficiency of protein transfer was assessed by Ponceau S staining of the membrane and Coomassie brilliant blue staining of the gel. Blots were blocked with 1% bovine serum albumin (BSA) and 3% nonfat dry milk in phosphate-buffered saline (PBS; 137 mM NaCl, 2.68 mM KCI, 10 mM Na₂HPO₄, 1.76 mM KH₂PO₄, pH 7.4) and incubated overnight at 4°C with primary antibodies diluted in 0.5% Tween-20 in PBS. Then, they were washed and incubated for 1 hr at ambient temperature with horseradish peroxidase-conjugated secondary antibodies (Boehringer Mannheim, Indianapolis, IN). The following secondary antibodies were used: anti-rabbit at 1:40000 and anti-mouse at 1:5000. Chemiluminescence was detected using the SuperSignal kit (Pierce, Evansville, IL) and documented on BioMax Light films (Kodak, Rochester, NY). The following primary antibodies were used for immunoblotting: calbindin (mouse #C9848 Sigma, 1:3000), synaptophysin (mouse #S5768, Sigma, 1:2000), recoverin (rabbit #AB5585, Chemicon, 1:5000), microtubule-associated protein 2 (mouse #M4403, Sigma, 1:500), NF200 (Rabbit #N4142, Sigma, 1:1000), tubulin (mouse T9026, Sigma, 1:5000), histone H3 (rabbit # AB1791, Abcam, 1:5000), PSD95 (mouse # 05-494 Upstate biochemicals, 1:10000), ABCA1 (rabbit #AB7360 Abcam, 1:1000), ApoA1 (rabbit #K23500R, Biodesign, 1:2000), RXRα (D20, Sigma, 1:200), NPC1 (rabbit #400148 Novus, 1:2000).

Annexed figure 4 shows the experimental results obtained by using above-disclosed protocol B.1. Immunoblots of retinae from wildtype and NPC1 mutant mice that were reacted with selected antibodies. In figure 4, WT = "wild type" and NPC = "NPC mice".

Protein levels of neuronal markers (calbindin, recoverin) and of cytoskeletal (NF200, MAP2, tubulin) and synaptic components (synaptophysin, PSD95, synaptogyrin and GRIP) were compared on protein concentration-adjusted samples from retinae of two months old wildtype and and NPC1 mutant mice.

As shown in figure 4, NPC1-deficient mice showed characteristic changes in protein levels compared to wildtype controls. This included a decreased level of calbindin, which is expressed by subtypes of retinal neurons including ganglion cells, whereas the level of recoverin, which is expressed by photoreceptors, did not change. Of the neuritic components, NPC1 mutant retinae showed a strong decrease in the dendritic marker Map2, but no changes in tubulin or the axonal marker neurofilament 200 indicating the selective degeneration of dendrites. Of the synaptic markers tested, the synaptic vesicle component synaptophysin was strongly reduced in NPC animals (figure 4), whereas the level of the postsynaptic component PSD95 was similar in wildtype and mutant mice.

The accumulation of cholesterol in the RPE and GCL prompted the inventors to examine the levels of components involved in cholesterol homeostasis.

Control immunoblots confirmed absence of NPC1 protein in NPC mice and its presence in wildtype animals (figure 4). Among the components involved in cholesterol homeostasis, notably ApoA1 and ABCA1 were increased in retinae from NPC mice compared to those from wildtype littermates. The upregulation of these proteins indicated activation of specific nuclear receptors, namely the liver X/retinoic acid X receptor (LXR/RXR), which are known to control these components at the transcription level. Accordingly, retinae from NPC mice contained a higher content of RXR alpha subunit that may further enhance signaling by LXR/RXR heterodimers (see figure 4).

Histone H3 expression was used as loading control. Immunoblot samples represent material from 3 animals (6 retinae). Experiments were repeated 3 times with littermates as a WT control.

These experimental results indicate that NPC-deficient mice are usable as models for AMD according to the present invention.

### Example 2 Screening method using a mouse-based experimental model

The animal model is based on our finding that NPC1 deficiency in the retina causes histo- and physiopathological changes that mimick symptoms of AMD as presented in example 1.

The animal model allows to test, whether drug candidates for therapy of AMD can revert the pathologic changes. Different models of retinal NPC1-deficiency can be explored based on the present invention.

First, the classic mouse model for NPC, which carries a spontaneous mutation in NPC1 that renders the protein non-functional, and second, transgenic mouse lines where NPC1 can be eliminated selectively in specific retinal cells by conditional gene ablation using the Cre/loxP system.

### Protocol for preclinical drug screening on NPC1 deficient mice as experimental model

Adult (two months old) mice with retinal deficiency in NPC1 are treated with test substances at different doses and examined at different time periods after drug administration. Depending on their solubility and blood-brain barrier penetrance, substances are administrated orally, by intraperitoneal injection or - by intraocular injections. Drug administration via intraocular injections can avoid possible systemic drug effects. Two modes of intraocular injections are possible, either intravitreal or subretinal. The latter type primarily targets RPE cells [Ref. 19].

The protocol for intraocular injections follows published procedures [Ref. 20]. For intravitreal or subretinal injections, adult mice are first anaesthetized using intraperitoneal injection of a solution containing an alpha-2 agonist (Xylazine, 5-12 mg/kg) and a dissociating anaesthetic (Ketamine 50-70 mg/kg). A topical anaesthetic (e.g. proparacain at 0.5%) is applied to the eye. The pupils are dilated by eye drops containing a mix of tropicamide (1%) and phenylephrine (2.5%). Ocular injections are performed under an ophthalmic surgical microscope or a dissecting microscope. A tiny incision is made in the peripheral cornea using a microscalpel or a bevelled 28 gauge hypodermic needle. Injections of test substances dissolved in suitable solutions or vehicle are then made with a 33-gauge blunt-ended hypodermic needle fitted on a Hamilton syringe (10 µl). The needle is inserted toward the back of the eye avoiding trauma to the lens and iris. Injections are intravitreal, before the needle reaches the retina and subretinal, if the needle is pushed until a slight resistance is felt. Injected volumes should not exceed 1.0 µl and should be injected slowly within approximately 30s. To control for successful injections, 1% fluorescein is added to to injection solution. Subretinal injections can be recognized by subretinal blebs, whereas intravitreal injections are indicated by widespread fluorescence filling the eye.

Immediately after injection, a drop of 1% atropine and a small amount of an ophthalmic ointment containing an antibiotic mixture is applied to the eye (e.g. Neomycin & Polymyxin B Sulfates & Dexamethason Ophthalmic Ointment) to reduce the risk of infection. This post-operative care of drops and ointment is administered once/day for the first 3 days.

Depending on their bioavailability, substances are administered singularly or repeatedly to maintain bioactive concentrations. Repeated administrations are only possible for oral and intraperitoneal, but not for intraocular injections.

Experimental controls include age-matched wildtype animals treated with test substances as well as vehicle-injected NPC1 mutant mice. Tests are performed on groups of defined sex.

After different periods of treatment, the histological and functional properties of retinae of mice from the test and control group are analysed using histochemical or immunohistochemical staining of retinae from mice fixed by transcardial perfusion with formaldehyde and for ERG recordings from anesthetized mice [Ref. 21] following standard procedures.Test substances are considered efficient, if they can revert or abolish the observed histopathological changes in NPC1 mutant animals, and if they are equally or more efficient than reference drugs that have proven and established effects in preclinical or clinical tests on AMD.

### Example 3 illustrating the cell-based experimental model: how to obtain RPE cells and OS from NPC1-deficient mice

The cell-based assay for preclinical screening for AMD-related drugs is based on the hypothesis that the AMD-related symptoms in NPC1 mutant mice are caused by defective phagocytosis of photoreceptor OS by RPE cells due to absence of NPC1. This assay requires RPE cells and photoreceptors that lack NPC1.

The following protocol can be used to isolate RPE cells from mouse eyes. It follows published procedures [Ref. 22, 23]. To isolate RPE cells, mice are killed by cervical dislocation. Eyes are enucleated and a circumferential incision is made above the ora serrata, to remove the cornea, lens, iris, and vitreous body. Enucleated eyecups are treated with 1 mg/ml bovine hyaluronidase (Sigma) in Ca2+-Mg2+-free Hepes-buffered Hanks' saline for 45 min to allow peeling off the neural retina and expose the RPE. After incubation in 2 mg/ml trypsin in Hepes-buffered Hanks' saline for 45 min, patches of RPE are peeled off manually with needles from the underlying choroid and then seeded on serum-coated glass coverslips and grown in defined medium.

Photoreceptor OS are isolated according to published procedures [Ref. 24, 25].

Mice are killed, retinae are removed, transferred to isolation medium (20% sucrose, 20 mM Tris-Cl, 2 mM MgCl₂, 130 mM NaCl, pH 7.2; 9-13 retinas/ml) and carefully homogenized on ice in phosphate buffer containing Complete protease inhibitor cocktail (Roche, Switzerland; 1 tablet in 50 ml homogenate). The homogenate is then subjected to density-gradient centrifugation (140000g, 70 min, 4°C, Beckman XL80 ultracentrifuge) using a sucrose-gradient [20%, 27%, 33%, 50% and 60% (w/v)]. The gradient is centrifuged at 185,000 g for 1 hr at 4 C.

Three orange-colored bands that contain ROS are collected from each of the gradients, pooled, and diluted 1:3 with BSS (balanced salt solution; 10 mM HEPES, 137 mM NaCl, 5.36 mM KCI, 0.81 mM MgSO₄, 1.27 mM CaCl₂, 0.34 mM Na₂HPO₄,0.44 mM KH₂PO₄, pH 7.4-7.5).

The rod OS are pelleted by centrifugation at 7700g for 10 min at 4 C, resuspended in several ml of BSS, and repelleted at 800g for 10 min. Protein content of the OS-containing fraction is determined by means of the Bradford assay.

### Example 4 Example for chemical treatment to obtain a cell-based model for AMD.

Previous studies have shown that treatment of cultured cells with specific chemicals including 3-beta-[2-(diethylamino)ethoxy]androst-5-en-17-one (also called U18666A) or A2E can induce very similar changes in intracellular cholesterol transport as deficiency in NPC1 [Ref 11, 12, 17]. This allows for alternative versions of a cell-based experimental model for AMD that is based on pharmacological treatment of RPE cells and therefore independent from NPC1 deficiency.

The model is prepared according to the following protocol, which follows published procedures for treatment with U18666A [Ref. 26]. To induce the "NPC-like" phenotype, rodent RPE cells are incubated for at least 24 hrs with 1-2 µM U18666A in the presence or absence of low density lipoprotein (LDL) particles. Notably, the pharmacological induction of the NPC-like defect also allows to use cell lines that show similar properties as RPE cells, e. g. the human line ARPE-19 [Ref. 27] (CRL-2302 or CRL-2502; American Type Culture Collection).

### Example 5: Screening method using a cell-based experimental model

The cellular model to test drug candidates for the therapy of AMD is based on the hypothesis that the AMD-related phenotype in NPC1 mice is caused by defects in the regeneration of photoreceptor OS. This involves phagocytosis of OS by RPE cells and their subsequent regeneration by photoreceptors. The first part of this process can be mimicked by an in vitro model of OS phagocytosis according to published protocols [Ref. 22, 28].

Cells used for the screen can be RPE cells isolated from wildtype and NPC1-deficient mice (see protocol C.1. as described in Example 3). As alternative to knockout mice, NPC1 deficiency can be induced by treatment of RPE cultures derived from wildtype mice (or rats) with NPC1-specific siRNA [Ref. 10]. The cellular phenotype of NPC1-deficiency can also be induced by pharmacological treatment (see Example 4).

For the preclinical screening, cultures of RPE cells or cell lines are prepared following a published protocol [Ref. 28]. If cell-lines are used, cells are first plated onto gelatine-coated 25-ml flasks with medium F99 (1:2 dilution of medium 199 and Ham's F12; Gibco Invitrogen) supplemented with 10% (v/v) FCS (Biochrom), 1 mM sodium pyruvate, 1 µg bovine insulin /ml (Sigma), and 50 µg gentamycin/ml and 2.5/ml µg amphotericin B. Cells are grown to subconfluence at 33°C and 5% CO₂ in air) and passaged using a trypsin /EDTA solution (0.05%/0.02%; Invitrogen) with a split ratio of 1:4.

First, effects of test substances on the viability of cells in the presence and absence of OS are determined after 48 to 72 hrs of treatment using the Live-Dead assay (Molecular Probes/Invitrogen), which is performed according to manufacturers instructions. This test is crucial and must be performed first: if a substance diminishes the viability of RPE cells or alike, it will not be used for the subsequent phagocytosis assay.

Phagocytosis of OS by RPE cells is measured using a published procedure [Ref. 28]. To this end, 10000 cells per well from the expanded cell line or primary cells, are plated on gelatine-coated 96-well plates and cultured for 24 hrs in F99 supplemented with 10% fetal bovine serum. If serum-free conditions are required, cells can be cultured in endothelial serum-free medium (Invitrogen). Medium is changed at 24 hrs. After 48 to 72 hrs in culture, DAPI nucleic stain is applied and the number of cells per well is measured using a fluorescence multi-well plate reader at 360 ± 40 nm excitation and 460 ± 40 nm emission.

For the phagocytosis assay, OS are labelled with 100 µg SNAFL-2 (Molecular Probes/Invitrogen) per 1 mg OS protein. The dye allows to evaluate OS binding (yellow-orange) and ingestion (green) separately, based on pH-dependent fluorescence properties. OS are added at a concentration of 5×10⁶ per 100 µl to each well (1.5×10⁷ OS/cm2) with fresh medium.

Cell cultures are incubated at 37°C for 6 hrs, rinsed twice with 50 µl PBS (with Ca2+/Mg2+) per well and then rinsed twice with 50 µl PBS (with Ca2+/Mg2+) containing 10% glycerol at pH 9.0. Binding of OS (excitatory wavelengths at 560±10 nm and recording emitted wavelengths at 645 ± 20 nm) and uptake of OS (excitatory wavelengths at 485 ± 10 nm and recording emitted wavelengths at 580±25 nm) are detected using a fluorescence multi-well plate reader.

Cellular autofluorescence is determined for each test by analyzing cells from wells to which no OS had been added. Mean cellular autofluorescence is subtracted from experimental values to determine the experimental fluorescence resulting from each variable. Measurements are performed in triplicates.

For both assays, controls include first addition of suitable vehicle (solvent for test substances) to cultures of NPC1-deficient cells or cells treated pharmacologically to induce the NPC phenotype. Second, addition of test substances to wildtype RPE cells or cells and cell lines that were not treated with pharmacological agents to induce the NPC phenotype.

To figure as potential drug candidate for AMD, a test substance has to leave viability of RPE cells unaffected and to revert a defect in outer segment phagocytosis by RPEs due to NPC1 deficiency or due to genetic or pharmacological treatment that mimicks NPC1 deficiency.

The experiments described above establish NPC1-deficiency in mice as model for late stages of preclinical development, mainly for pharmacological studies of drug candidates.

### Bibliography

**[Ref. 1]** Rattner, A., and J. Nathans. 2006. Macular degeneration: recent advances and therapeutic opportunities. Nat. Rev. Neurosci. 7: 860-872.
**[Ref. 2]** de Jong, P. T. 2006. Age-related macular degeneration. N. Engl. J. Med. 355: 1474-1485.
**[Ref.3]** Rakoczy E.P., M. J. Yu, S. Nusinowitz, B. Chang, and J. R. Heckenlively. 2006. Mouse models of age-related macular degeneration. Exp. Eye Res. 82: 741-752.
**[Ref. 4]** Pentchev, P. G., A. E. Gal, A. D. Booth, F. Omodeo-Sale, J. Fouks, B. A. Neumeyer, J. M. Quirk, G. Dawson, and R. O. Brady. 1980. A lysosomal storage disorder in mice characterized by a dual deficiency of sphingomyelinase and glucocerebrosidase. Biochim. Biophys. Acta 619: 669-679.
**[Ref.5]** Loftus, S. K., J. A. Morris, E. D. Carstea, J. Z. Gu, C. Cummings, A. Brown, J. Ellison, K. Ohno, M. A. Rosenfeld, D. A. Tagle, P. G. Pentchev, and W. J. Pavan. 1997. Murine model of Niemann-Pick C disease: mutation in a cholesterol homeostasis gene. Science 277: 232-235.
**[Ref. 6]** Sauer, B. 1998. Inducible gene targeting in mice using the Cre/lox system. Methods 14: 381-392.
**[Ref. 7]** Ohtsubo, K., and J. D. Marth. 2007. Cre Recombinase Gene Transfer In Vitro and Detection of loxP-Dependent Recombination. CSH Protocols 2007; doi:10.1101/pdb.prot4761.
**[Ref. 8]** Mori, M., D. Metzger, J. M. Garnier, P. Chambon, and M. Mark. 2002. Site-specific somatic mutagenesis in the retinal pigment epithelium. Invest Ophthalmol. Vis. Sci. 43: 1384-1388.
**[Ref. 9]** Jimeno, D., L. Feiner, C. Lillo, K. Teofilo, L. S. Goldstein, E. A. Pierce, and D. S. Williams. 2006. Analysis of kinesin-2 function in photoreceptor cells using synchronous Cre-IoxP knockout of Kif3a with RHO-Cre. Invest Ophthalmol. Vis. Sci. 47: 5039-5046.
**[Ref. 10]** Chinetti-Gbaguidi, G., E. Rigamonti, L. Helin, A. L. Mutka, M. Lepore, J. C. Fruchart, V. Clavey, E. Ikonen, S. Lestavel, and B. Staels. 2005. Peroxisome proliferator-activated receptor alpha controls cellular cholesterol trafficking in macrophages. J. Lipid Res. 46: 2717-2725.
**[Ref. 11]** Liscum, L., and J. R. Faust. 1989. The intracellular transport of low density lipoprotein-derived cholesterol is inhibited in Chinese hamster ovary cells cultured with 3-beta-[2-(diethylamino)ethoxy]androst-5-en-17-one. J. Biol. Chem. 264: 11796-11806.
**[Ref. 12]** Roff, C. F., E. Goldin, M. E. Comly, A. Cooney, A. Brown, M. T. Vanier, S. P. Miller, R. O. Brady, and P. G. Pentchev. 1991. Type C Niemann-Pick disease: use of hydrophobic amines to study defective cholesterol transport. Dev. Neurosci. 13: 315-319.
**[Ref. 13]** Pentchev, P. G. 2004. Niemann-Pick C research from mouse to gene. Biochim. Biophys. Acta 1685: 3-7.
**[Ref. 14]** Claudepierre, T., and F. W. Pfrieger. 2003. [New aspects of cholesterol in the central nervous system]. Med. Sci. (Paris) 19: 601-605.
**[Ref. 15]** Pfrieger, F. W. 2003. Outsourcing in the brain: do neurons depend on cholesterol delivery by astrocytes? Bioessays 25: 72-78.
**[Ref. 16]** Curcio, C. A., J. B. Presley, G. Malek, N. E. Medeiros, D. V. Avery, and H. S. Kruth. 2005. Esterified and unesterified cholesterol in drusen and basal deposits of eyes with age-related maculopathy. Exp. Eye Res. 81: 731-741.
**[Ref.17]** Lakkaraju, A., S. C. Finnemann, and E. Rodriguez-Boulan. 2007. The lipofuscin fluorophore A2E perturbs cholesterol metabolism in retinal pigment epithelial cells. Proc. Natl. Acad. Sci. U. S. A 104: 11026-11031.
**[Ref. 18]** Johnson, P. T., M. N. Brown, B. C. Pulliam, D. H. Anderson, and L. V. Johnson. 2005. Synaptic pathology, altered gene expression, and degeneration in photoreceptors impacted by drusen. Invest. Ophthalmol. Vis. Sci. 46: 4788-4795.
**[Ref. 19]** Kachi, S., Y. Oshima, N. Esumi, M. Kachi, B. Rogers, D. J. Zack, and P. A. Campochiaro. 2005. Nonviral ocular gene transfer. Gene Ther. 12: 843-851.
**[Ref. 20]** Timmers, A. M., H. Zhang, A. Squitieri, and C. Gonzalez-Pola. 2001. Subretinal injections in rodent eyes: effects on electrophysiology and histology of rat retina. Mol. Vis. 7: 131-137.
**[Ref. 21]** Peachey, N.S., S.L. Ball SL (2003) Electrophysiological analysis of visual function in mutant mice. Doc. Ophthalmol. 107: 13-36.
**[Ref. 22]** Nandrot, E. F., Y. Kim, S. E. Brodie, X. Huang, D. Sheppard, and S. C. Finnemann. 2004. Loss of synchronized retinal phagocytosis and age-related blindness in mice lacking alphavbeta5 integrin. J. Exp. Med. 200: 1539-1545.
**[Ref. 23]** Gibbs, D., and D. S. Williams. 2003. Isolation and culture of primary mouse retinal pigmented epithelial cells. Adv. Exp. Med. Biol. 533: 347-352.
**[Ref. 24]** Chaitin, M. H., and M. O. Hall. 1983. Defective ingestion of rod outer segments by cultured dystrophic rat pigment epithelial cells. Invest Ophthalmol. Vis. Sci. 24: 812-820.
**[Ref. 25]** Papermaster, D. S. 1982. Preparation of retinal rod outer segments. Methods Enzymol. 81: 48-52.
**[Ref. 26]** Higgins, M. E., J. P. Davies, F. W. Chen, and Y. A. Ioannou. 1999. Niemann-Pick C1 is a late endosome-resident protein that transiently associates with lysosomes and the trans-Golgi network. Mol. Genet. Metab 68: 1-13.
**[Ref.27]** Dunn, K. C., A. E. Aotaki-Keen, F. R. Putkey, and L. M. Hjelmeland. 1996. ARPE-19, a human retinal pigment epithelial cell line with differentiated properties. Exp. Eye Res. 62: 155-169.
**[Ref. 28]** Karl, M. O., M. Valtink, J. Bednarz, and K. Engelmann. 2007. Cell culture conditions affect RPE phagocytic function. Graefes Arch Clin. Exp. Ophthalmol. 245: 981-991.

## Claims

1. Use of an animal or a cell that is deficient in functional Niemann Pick type C-related protein as experimental model of age-related macular degeneration (AMD) and vision disorders resulting from AMD.

2. The use of claim 1, wherein said animal or cell shows accumulation of lipids in the endosomal-lysosomal system.

3. The use of claim 2, wherein said accumulation is generated by a mutation in a NPC gene selected from the group comprising NPC1, NPCL1 and NPC2 genes.

4. The use of claim 2, wherein said accumulation is induced by mutation of encoding DNA or by down regulation of mRNA.

5. The use according to anyone of claims 1-4, wherein said animal is a non-human primate.

6. The use according to anyone of claims 1-4, wherein said animal is a mouse.

7. The use according to claim 6, wherein said mouse is a mutant homozygote NPC1 mouse.

8. The use according to claim 6, wherein said mouse is a mouse with deficiency in functional NPC1 due to transfection of siRNA by virus or direct injection.

9. The use of claim 6, 7 or 8, wherein said mouse is a BALB/cNctr-Npc1^{m1N}/J mouse.

10. The use according to any one of claims 1-4, wherein said cell is a retinal pigment epithelium cell or a photoreceptor.

11. The use of claim 10, wherein the cell is a cell line obtained from a mutant homozygote NPC1 mouse.

12. The use according to claim 10 or 11, wherein said cell is obtainable by treating respectively retinal pigment epithelium cells or photoreceptors with 3-béta-[2-diéthylamino)éthoxy]androst-5-en-17-one).

13. The use according to anyone of claims 1-12, wherein the AMD model is a model for all forms of AMD.

14. The use according to anyone of claims 1-12 in a screening method of molecule candidate for the treatment of AMD.
